(19) Europäisches Patentamt
European Patent Office
Office européen des brevets

(11) Publication number: **0 003 644**
**A1**

(12) **EUROPEAN PATENT APPLICATION**

(21) Application number: **79300116.5**

(22) Date of filing: **23.01.79**

(51) Int. Cl.[2]: **C 12 D 9/00**, C 12 D 9/14, C 07 H 15/20, C 07 G 11/00, C 07 H 15/22, A 23 K 1/17

(30) Priority: **02.02.78 US 874306**
**02.02.78 US 874307**
**02.02.78 US 874308**
**02.02.78 US 874432**

(43) Date of publication of application: **22.08.79**
**Bulletin 79/17**

(84) Designated Contracting States: **BE CH FR GB NL SE**

(71) Applicant: **AMERICAN CYANAMID COMPANY, Berdan Avenue, Wayne New Jersey 06904 (US)**

(72) Inventor: **Tobkes, Martin, 98 Sutin Place, Spring Valley New York 10977 (US)**
Inventor: **Ingle, Donald Lee, 19 Holt Circle, Trenton New Jersey (US)**
Inventor: **Dann, Murray, 150 Prospect Avenue, Pearl River New York 10965 (US)**

(74) Representative: **Allam, Peter Clerk et al, TREGEAR, THIEMANN & BLEACH Enterprise House Isambard Brunel Road, Portsmouth, Hants PO1 2AN (GB)**

(54) **Animal feed supplements containing antibiotic trans-BM 123-gamma and process for making same.**

(57) The invention relates to animal feed supplements containing antibiotic trans-BM123. Such supplements may be economically obtained by adding to a fermentation whole harvest mash containing antibiotic trans-BM123, or to a filtered fermentation broth containing antibiotic BM123, at a suitable acid pH, a complexing agent selected from synthetic tanning agents, alkali metal alkyl sulfates and dioctyl sulfosuccinate salts, whereby to precipitate out a complex of the antibiotic with said complexing agent; and recovering the precipitated complex. These complexes aid in increasing the growth rate and feed efficiency when fed to arm animals, either alone or in combination with carriers.

EP 0 003 644 A1

Amendments requested by letter of March 5, 1979, received on March 8, 1979

Title: ANIMAL FEED SUPPLEMENTS CONTAINING ANTIBIOTIC TRANS-BM123γ AND PROCESS FOR MAKING SAME.

This invention relates to a method of recovering antibiotic BM123γ from fermentation whole harvest mashes containing it. More particularly, the novel process involves adding a synthetic tanning agent, an alkali metal alkyl sulfate or a dioctyl sulfosuccinate either to the whole harvest mash or to the filtered fermentation liquor, and recovering the so precipitated antibiotic complex by any convenient means. The invention also relates to the use of these and related complexes and salts in animal feed supplement compositions for enhancing the growth rate of animals such as poultry, swine, early weaned pigs, and ruminants such as cattle, sheep and goats.

Antibiotic *trans*-BM123γ may be formed by fermentative biosynthesis during the cultivation under controlled conditions of certain strains of an undetermined species of *Nocardia*, which are available from the Agricultive Research Service Culture Collection, Northern Regional Research Laboratory, Peoria, Illinois, U.S.A. where they are deposited under Nos. NRRL 5646, (deposited 27th September 1972) NRRL 8050 and NRRL 11230. Mutants of such strains may

deposited 3rd October, 1974 — deposited 22nd November, 1977.

also produce the antibiotic. The preparation and properties of antibiotics trans-BM123$\gamma_1$, trans-BM123$\gamma_2$, and trans-BM123 are set forth in U.S. Patent No. 4,007,167. Hereinafter, the term "trans-BM123$\gamma$" refers to a mixture in any proportions of trans-BM123$\gamma_1$ and trans-BM123$\gamma_2$.

The problem of recovering the antibiotic economically has been a serious one. In the patent referred to above, adsorption on carbon followed by elution and column chromatography are employed. Such a process is not excessively expensive where pure antibiotic is required for medical usage. However, when the antibiotic is to be used in animal feed supplement compositions the factor of cost is a very serious matter and there is, therefore, a need for an inexpensive process of recovering the antibiotic for this purpose.

The present invention deals with a process and in a more specific aspect also with a product. The process involves the precipitation of the antibiotic either from the whole harvest mash or from the filtered fermentation broth by the addition of a synthetic tanning agent, an alkali metal alkyl sulfate or a dioctyl sulfosuccinate salt.

More specifically, in its broadest aspect, the present invention provides a process for the production of an animal feed supplement, characterized by adding to a fermentation whole harvest mash containing containing antibiotic trans-BM123$\gamma$, or to a filtered fermentation broth containing antibiotic BM123$\gamma$, at a

suitable acid pH, a complexing agent selected from synthetic tanning agents, alkali metal alkyl sulfates and dioctyl sulfosuccinate salts, whereby to precipitate out a complex of the antibiotic with said complexing agent; and recovering the precipitated complex.

The synthetic tanning agent operable in the process of the present invention is a sulfited phenol formaldehyde condensate which may be represented by the following general formula:

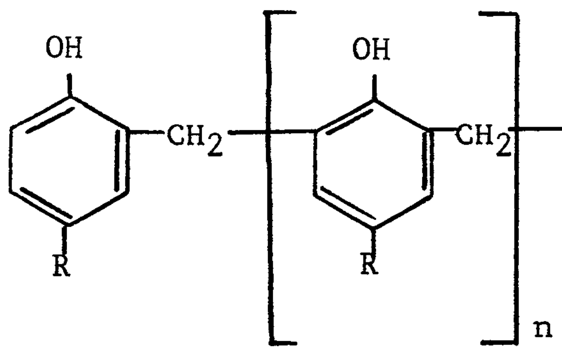

wherein R is hydrogen or a methylene sulfonic acid group ($-CH_2SO_3H$) and n is 0,1,2,3, or 4 with the proviso that about half of the R groups present are methylene sulfonic acid groups. This synthetic tanning agent is not a pure chemical compound but of necessity is obtained as a mixture having an estimated molecular weight of 420-530. It is readily prepared by first condensing phenol and formaldehyde in aqueous media followed by reaction of the intermediate condensate with formaldehyde, various sulfites, and buffer acids thus forming methylene-sulfonic acid groups ($CH_2SO_3H$) in the molecule. The product is an amorphous water soluble

material that may be obtained either in concentrated water solution or in powder form, and ranges from colorless to dark brown. In order to avoid cumbersome language, this synthetic tanning agent will be referred to by its generic name in the art as "syntan", and this term will be used extensively in the specification and appended claims. A sulfited phenol formaldehyde syntan of the above general type is sold by A.J. & J.O. Pilar Inc. of Newark, N.J. under the trademarks of TruTan® RT Regular and TruTan® RT New.

The alkali metal alkyl sulfates alternatively operable as complexing agent the process of the present invention may be represented by the following general formula:

$$CH_3-(CH_2)_n-O-SO_2-OM$$

wherein n is an integer from 9 to 17, inclusive, and M is sodium or potassium. Typical of such alkali metal alkyl sulfates which may be employed are, for example, sodium decyl sulfate, potassium hendecyl sulfate, sodium lauryl sulfate, potassium tridecyl sulfate, sodium myristyl sulfate, potassium pentadecyl sulfate, sodium cetyl sulfate, potassium haptadecyl sulfate, and sodium octadecyl sulfate. Mixtures of alkali metal alkyl sulfates may also be employed such as a mixture of sodium cetyl sulfate and sodium oleyl sulfate; a mixture of sodium hendecyl sulfate and potassium octadecyl sulfate; a mixture of potassium decyl sulfate and sodium heptadecyl sulfate; a mixture of

potassium lauryl sulfate and potassium cetyl sulfate; a mixture of sodium tridecyl sulfate, potassium myristyl sulfate, and sodium pentadecyl sulfate, and the like. When mixtures of alkali metal alkyl sulfates are employed, then a corresponding mixture of antibiotic-alkyl sulfate complexes are obtained.

The dioctyl sulfosuccinate salts operable in the process of the present invention are those prepared from sulfobutanedioic acid, 1,4-bis(2-ethylhexyl)ester and the alkali metal or alkaline earth metal cations. Typical of such salts are dioctyl sodium sulfosuccinate, dioctyl potassium sulfosuccinate, dioctyl calcium sulfosuccinate, dioctyl magnesium sulfosuccinate, etc. The preparation and properties of these dioctyl sulfosuccinate salts are set forth in U.S. Patent Nos. 2,028,091, 2,176,423, and 3,035,973. Mixtures of dioctylsulfosuccinate salts may also be employed such as a mixture of dioctyl lithium sulfosuccinate and dioctyl barium sulfosuccinate. When mixtures of dioctyl sulfosuccinate salts are employed, then a corresponding mixture of antibiotic-complexes are obtained.

The novel process of the present invention provides almost complete removal of the antibiotic activity from the fermentation mash or broth. Furthermore, the antibiotic-complexes so obtained can be used without separation of the constituents in animal feed supplement compositions, which is an important economic advantage. Therefore, in one of the aspects of the present invention the complex of

antibiotic trans-BM123$\gamma$ and the above-described compounds are included as new products.

The products of the antibiotic have been referred to as reversible antibiotic complexes. Their exact chemical nature has not been determined, but covalent bonding is not involved and the product is not a physical mixture.

The complex, derived from the interaction of the antibiotic and the syntan, alkali metal alkyl sulfate or dioctyl sulfosuccinate salt is not necessarily combined in any limiting stoichiometry. The chemical bonds are reversible since antibiotic trans-BM123$\gamma$ may be recovered from the complex by adsorption on a cross-linked carboxymethyldextran gel column followed by elution with aqueous acid. While it is not intended to limit the present invention to theories of chemical constitution and the like, it seems probable that the complexes of the present invention are sufficiently reversible so that under conditions of use in animal feed supplement compositions the antibiotic is set free upon ingestion.

As starting material for the novel processes of the present invention there may be employed the whole harvest mash obtained after completion of a fermentation with the aforementioned *Nocardia* sp. NRRL 5646, NRRL 8050, NRRL 11230 or mutants thereof. Preferably, there is employed the fermentation liquor or broth which has been clarified by removing the mycelia and other insolubles by filtration. Diatomaceous earth or any other conventional filtration aid may be used to assist in the filtration.

In the case of the syntan complex, the following procedure is preferably followed. The pH of the whole mash or the filtered broth may be first adjusted to between 1.8 and 5.0 with dilute aqueous acid. Suitable acids for this purpose may be, for example, dilute hydrochloric acid, dilute sulfuric acid, dilute trifluoroacetic acid, etc., although even glacial acetic acid may be used. Then an aqueous solution of the syntan is added slowly, with stirring, at ambient temperatures. The pH may then be adjusted at this point to 1.8-5.0 by acidifying as above or basifying with a suitable base such as aqueous ammonia or soda ash. The antibiotic and the syntan form a complex which is water insoluble and thus precipitates. The precipitated syntan complex or, in the case of the whole mash, the precipitated syntan complex together with the fermentation mash solids, is then removed by filtration or centrifugation and dried. The products so obtained may be dried by (1) slurrying the wet solids in polar, water miscible non-solvents such as acetone followed by filtration, rinsing and air-drying; or by (2) reslurrying the wet solids in water and freeze drying or spray drying.

When the products of the present invention are thus carefully dried under temperature conditions which do not degrade antibiotic trans-BM123 , they are usually white to tan powders in the case of the syntan complex. In the case of the syntan complex associated with dried harvest mash solids, they are usually gray to brown powders or solids. In the dry form, these products

are extremely stable, keeping without significant loss of antibiotic activity for considerable periods of time. This long storage life is, of course, an important practical advantage.

It is an advantage of the present invention that the amount of syntan added to precipitate the complex is not critical and no exact stoichiometric relations need to be followed. In general, the amount of syntan used will be somewhat in excess of the minimum required to form the complex with the antibiotic. Excess syntan will merely remain in solution upon filtration. The amount of syntan required is, however, directly proportional to the antibiotic concentration in the mash or liquor. The specific bioactivity of the precipitated complex also varies and it is in fact likely that the complex has varying relative amounts of antibiotic and, of course, is quite likely to be a mixture of complexes because the syntan used is not a pure single chemical compound.

The minimum amount of syntan required to form the complex with the antibiotic in any particular fermentation batch may be readily determined as follows. A sample (conveniently 50-100 mL) of the fermentation whole harvest mash is taken and clarified by removing the mycelia and other insolubles by filtration, preferably with a filter aid. The filtrate is then acidified to a pH of 1.805.0 with dilute aqueous mineral acid such as dilute hydrochloric acid, dilute sulfuric acid, dilute phosphoric acid, or the like. The solution is then titrated with the particular aqueous solution of

syntan which is to be used until no further precipitate or turbidity forms. The amount of syntan solution for the fermentation batch is then calculated from the titer of the sample taken, providing also for a slight excess.

In the case of the alkyl metal alkyl sulfate, the preferred procedure is that which will now be described. The pH of the whole mash or of the filtered broth is first adjusted to between 1.9 and 2.1, preferably about 2.0, with an acid. Suitable acids for this purpose may be, for example, hydrochloric acid, sulfuric acid, trifluoroacetic acid, and the like, although even glacial acetic acid may be used. This pH adjustment appears to be critical since below pH 1.9 there appears to be degradation of antibiotic trans-BM123γ during drying of the filter cake even at 40°C. under vacuum. Then, an aqueous solution of an alkali metal alkyl sulfate (or a mixture of alkali metal alkyl sulfates) is added slowly, with stirring, at ambient temperatures. The entire process of the present invention is preferably carried out at from about 15°C. to about 30°C., conveniently at room temperature. The antibiotic and alkyl sulfate form a complex which is water insoluble and thus precipitates. The precipitated complex or, in the case of the whole mash, the precipitated complex together with the fermentation mash solids, is then removed by filtration or centrifugation and dried. The products so obtained may be dried by (1) slurrying the wet solids in polar, water miscible non-solvents such as acetone followed by filtration, rinsing and air-drying; or by (2) reslurrying the wet solids in water and freeze drying or spray drying.

In the use of the dioctyl sulfosuccinate complex, the preferred procedure is as will now be described. The pH of the whole harvest mash or the filtered broth is first adjusted to between 2.5 and 6.0 with dilute aqueous acid. Suitable acids for this purpose may be, for example, dilute hydrochloric acid, dilute sulfuric acid, dilute trifluoroacetic acid, etc., although even glacial acetic acid may be used. Then an aqueous solution of a dioctyl sulfosuccinate salt (or a mixture thereof) is added slowly, with stirring, at ambient temperature. Diatomaceous earth is added and the antibiotic and the dioctyl sulfosuccinate salt form a complex which is water insoluble and thus precipitates. The precipitated complex or, in the case of the whole mash, the precipitated complex together with the fermentation mash solids, is recovered by filtration or centrifugation and dried. The products so obtained may be dried by slurrying the wet solids in polar, water miscible liquids such as acetone (which do not act as solvents for the complex) followed by filtration, rinsing and air-drying or by reslurrying the wet solids in water and freeze drying or spray drying. When the products of the present invention are thus carefully dried under temperature conditions which do not degrade antibiotic <u>trans</u>-BM123$\gamma$, they are usually tan to brown colored solids.

The amount of dioctyl sulfosuccinate salt

added to precipitate the complex with the antibiotic is normally the minimum required to form the complex with the antibiotic. The amount of dioctyl sulfosuccinate salt required is, however, directly proportional to the antibiotic concentration in the mash or liquor. The specific bioactivity of the precipitated complex also varies and it is, in fact, likely that the complex has varying relative amounts of antibiotic.

The minimum amount of dioctyl sulfosuccinate salt required to form the complex with the antibiotic in any particular fermentation batch may be readily determined as follows. A sample (conveniently 50-100ml.) of the fermentation whole harvest mash is taken and clarified by removing the mycelia and other insolubles by filtration, preferably with a filter aid. The filtrate is then acidified to a pH of 2.5 to 4.8 with dilute aqueous mineral acid. This solution is then titrated with the particular aqueous solution of dioctyl sulfosuccinate salt which is to be used until no further precipitate or turbidity forms. The amount of dioctyl sulfosuccinate salt solution for the fermentation batch is then calculated from the titer of the sample taken.

The *trans*-BM123 -complexes which can be produced by the process of the present invention can be used as growth-promoting agents for farm animals. We have also found that antibiotic *trans*-BM 123$\gamma$ itself, salts thereof, and alkylated derivatives thereof, are similarly useful as growth-promoting agents for animals.

Thus, in another aspect, the present invention

provides an animal feed supplement characterized as a dried mixture of fermentation harvest mash solids and an anti bacterial ingredient selected from the group consisting of antibiotic trans-BM123γ, an antibiotic trans-BM123γ salt, an antibiotic trans-BM123γ alkyl sulfate complex, an antibiotic trans-BM123γ dioctyl sulfossuccinate complex, an antibiotic trans-BM123γ syntan complex, an antibiotic trans-BM123γ alkylated derivative, and mixtures thereof in any proportion.

The invention also provides an animal feed composition for improving feed efficiency and enhancing the growth rate of animals and poultry characterized as a nutritionally balanced animal feed containing from about 0.001% to about 1.0% by weight of the feed of an antibacterial ingredient selected from the group consisting of antibiotic trans-BM123γ, an antibiotic trans-BM123γ salt, an antibiotic trans-BM123γ alkyl sulfate complex, an antibiotic trans-BM123 dioctyl sulfosuccinate complex, an antibiotic trans-BM123 syntan complex, an antibiotic trans-BM123γ alkylated derivative, and mixtures thereof in any proportion.

The alkylated derivatives of trans-BM123 which are effective as growth promoting agents herein are described in U.S. Patent 4,048,431.

In accordance with the present invention, the dried complexes or the dried harvest mash solids containing the complexes, either along or in combination with suitable carriers, when added to an animal feed

aid in increasing the growth rate. In addition, feed efficiency is improved. The present invention has the advantage that the growth rate of non-ruminants such as poultry and swine and especially weanling pigs is significantly increased, and that feed conversion rates are noticeably enhanced.

The feed supplement compositions of the present invention suitably are administered in an amount sufficient to furnish approximately the following dosage levels in mg./head/day:

| | |
|---|---|
| Large ruminants | 350 |
| Small ruminants | 200 |
| Non-ruminants | 100 |
| Poultry | 2 |

The milligrams per pound of antibiotic trans-BM123$\gamma$ present in any particular supplement composition of the present invention may be readily determined by bioassay as set forth in U.S. Pat. No. 4,007,167. The preferred method is an adaptation of the *Staphylococcus aureus* turbidimetric assay for tetracycline that is described in the manual "Assay Methods of Antibiotics, a Laboratory Manual" by D.C. Grove and W.A. Randall, Medical Encyclopedia Inc. (1955) pages 48-52, substituting *Klebsiella pneumoniae* as the test organism. From the potency data thus obtained, the pounds of feed supplement composition to be used per ton of feed may be readily calculated.

A wide variety of carriers may be used in the preparation of the feed supplement compositions of this invention. Carriers suitable for use to make up the feed supplement compositions include the following:

soybean meal, alfalfa meal, cotton seed oil meal, linseed oil meal, cornmeal, cane molasses, urea, bone meal, corncob meal, and the like. The carrier promotes a uniform distribution of the complex in the finished feed into which the supplement is blended. It thus performs an important function by ensuring proper distribution of the complex throughout the feed.

The following examples illustrate the invention.

## Example 1

## Preparation of Antibiotic *trans*-BM123γ- Syntan Complex from Whole Harvest Mash

Thirty liters of *Nocardia* sp. NRRL 11230 fermentation mash containing 347 mcg. of *trans*-BM123γ antibiotic per ml. is used at harvest pH 5.7. A 750 ml. portion of Trutan® RT New is added slowly to the mash with stirring. The mixture is stirred for one hour, 600 g. of diatomaceous earth is added and the mixture is filtered. The filter cake is dried *in vacuo* at 40°C. for 48 hours, giving 1.5 kg. of dried material containing the antibiotic *trans*-BM123γ - syntan complex.

*Nocardia* sp. NRRL 11230 has cultural, physiological, and morphological characteristics essentially the same as those of NRRL 5646 and NRRL 8050; see e.g. U.K. Patent Nos. 1,536,153/4.

## Example 2

## Preparation of Antibiotic trans-BM123γ - Syntan Complex from Harvest Mash Filtrate

To 3 liters of stirred *Nocardia* sp. NRRL 11230 fermentation mash filtrate, assaying 475 mcg. of antibiotic *trans*-BM123γ per ml. is added 52.5 ml. of TruTan® RT New. The pH of the resultant slurry is adjusted to 4.75 with 6N hydrochloric acid, stirred for 5 minutes and allowed to settle for 45 minutes. The solids are recovered by filtration, washed with a small amount of water and dried *in vacuo*, without heat, giving 35.18 g. of product.

## Example 3

## Preparation of antibiotic BM123γ - lauryl sulfate complex from whole harvest mash

A 28 liter portion of *Nocardia* sp. NRRL 11230

fermentation mash containing 571 mcg. of antibiotic BM123γ per ml. is adjusted to pH 2.0 with dilute sulfuric acid. A 218 g. portion of sodium lauryl sulfate is added as a 5% aqueous solution and pH is readjusted to 2.0 with dilute sulfuric acid. The mixture is stirred for 45 minutes, 60g. of diatomaceous earth is added, and the mixture is filtered. The solid complex is dried *in vacuo* at 40°C. for 67 hours giving 1.4 kg. of material containing the antibiotic BM123γ - lauryl sulfate complex.

## Example 4.

### Preparation of antibiotic BM123γ - lauryl sulfate complex from harvest mash filtrate

To 6 liters of *Nocardia* sp. NRRL 11230 fermentation mash filtrate containing 447 mcg. of antibiotic BM123γ per ml. there is added with stirring 90.0 g. of diatomaceous earth followed by 420 ml. of 10% w/v aqueous sodium lauryl sulfate. The pH of the suspension is adjusted to 2.5 with 50% w/w sulfuric acid, stirred for 15 minutes and then filtered. The filter cake is rinsed with a small amount of water and then dried for three days *in vacuo* without heat, giving 223.4 g. of product assaying 4.9 mcg./mg.

## Example 5

### Preparation of antibiotic *trans*-BM123γ dioctyl sulfosuccinate complex from harvest mash filtrate

To a 40 liter portion of *Nocardia* sp. NRRL 11,230

fermentation mash filtrate containing 533 mcg. of antibiotic trans-BM123γ per ml., is added 800 g. of sodium chloride. The filtrate is adjusted to pH 4 with hydrochloric acid. A 600 g. portion of diatomaceous earth is added followed by one liter of an aqueous alcoholic solution of dioctyl sodium sulfosuccinate. The pH is readjusted to 4.0 with hydrochloric acid, 600 g. of diatomaceous ear h is added and the mixture is stirred for 15 minutes. The mixture is allowed to settle for one hour and the supernatant is syphoned off. The pH of the settled material is adjusted to 5 with sodium hydroxide. The mixture is then freeze dried giving 3.02 kg. of material containing antibiotic trans-BM123γ dioctyl sulfosuccinate complex.

Example 6

Preparation of antibiotic trans-BM123γdioctyl sulfosuccinate complex from harvest mash filtrate

To a 100 ml. portion of *Nocardia* sp. NRRL 11230 mash filtrate, assaying 582 mcg./ml. of antibiotic trans-BM123γ , is added with stirring 1.0 ml. of a 70% ethanolic solution of dioctyl sodium sulfosuccinate. The pH of the mixture is adjusted to 4.75 with 6N hydrochloric acid and the mixture is centrifuged. The supernatant is decanted and the precipitate is dried *in vacuo* for 4 days without heat to give 1.54 g. of product.

Example 7

Growth Promoting Effect of Antibiotic trans--BM123γ - HCL and Complexes on Poultry

One day old Hubbard X Hubbard crossbred chicks

were used. These chicks are randomly allotted to pens of ten chicks (5 male and 5 female) each. Four experiments are started at one week intervals. In each experiment, four pens of chicks are used for unmedicated controls and two pens of chicks are used at each level of drug. Thus, a total of 16 pens (160 chicks) are used as controls and a total of 8 pens (80 chicks) are used at each level of drug. The duration of each experiment is 13 days.

The controls are offered an unmedicated diet of broiler ration (composition follows) and water *ad libitum*. The medicated chicks are offered the same diet containing antibiotic *trans*-BM123γ -TruTan® RT New complex at levels of 1,2,5,10,20 and 30 parts per million and water *ad libitum*. The weight of the chicks is determined at the beginning and on completion of the experiments. Weight gain and the amount of feed consumed are also determined. The data are averaged and summerized in Table I to IV below, together with the percent improvement in weight gains and feed/gain ratios.

Brioler ration formula:

| Component | Percent by Weight |
|---|---|
| Ground yellow corn | 53.45 |
| Soybean oil meal (49%) | 28.00 |
| Menhaden fish meal (60%) | 5.00 |
| Corn gluten meal (60%) | 5.00 |
| Dehydrated alfalfa meal (17%) | 2.00 |
| Stabilized fat | 4.00 |
| Dicalcium phosphate | 1.20 |
| ground limestone | 0.50 |

| Broiler ration formula: | Percent by weight |
|---|---|
| Sodium chloride | 0.30 |
| Trace minerals mixture* | 0.05 |
| Vitamin premix** | 0.50 |

*Trace mineral mixture

| Component | | One lb./ton furnishes |
|---|---|---|
| Manganese | 12.50% | 62.5 ppm |
| Iron | 6.00% | 30.0 ppm |
| Zinc | 5.00% | 25.0 ppm |
| Copper | 0.65% | 3.25 ppm |
| Iodine | 0.35% | 1.75 ppm |
| Cobalt | 0.25% | 1.25 ppm |
| Calcium (min. 15.30%, max. 18.35%) | | |

**Vitamin premix for one ton

| Component | Weight in grams |
|---|---|
| DL. Methionine | 453.6 |
| Butylated hydroxy toluene | 113.6 |
| Vitamin A (30,000 mcg./g. | 100.0 |
| Vitamin $D_3$ (200,000 mcg./g.) | 5.0 |
| Vitamin E (20,000 mcg./lb.) | 45.4 |
| Riboflavin | 4.0 |
| Niacinamide | 25.0 |
| Calcium pantothenate | 8.0 |
| Vitamin K (menadione) | 1.0 |
| Folic acid (10%) | 13.0 |
| Choline chloride (50%) | 908.0 |
| Vitamin $B_{12}$ (20 mg./lb.) | 227.0 |
| Corn oil | 50.0 |
| Fine Ground corn | 2,582.4 |

TABLE I

| Treatment | Drug Level in Feed (ppm) | Average weight per chick in Gram | | Average weight gain per chick in Gram | Feed Consumed per chick in Gram (Average) | Feed/ Gain Ratio | Percent Improvement in | |
|---|---|---|---|---|---|---|---|---|
| | | Start | End | | | | Gain | Feed/Gain Ratio |
| Control | 0 | 44.0 | 239.3 | 195.4 | 292.1 | 1.495 | | |
| Antibiotic trans-BM123γ-hydrochloride mixture | 5 | 44.1 | 248.3 | 204.3 | 297.9 | 1.458 | 4.6 | 2.5 |
| | 10 | 44.0 | 246.4 | 202.5 | 293.6 | 1.450 | 3.6 | 3.0 |
| | 20 | 44.1 | 258.5 | 214.4 | 301.7 | 1.407 | 9.7 | 5.9 |
| Antibiotic trans-BM123γ Lauryl sulfate | 1 | 44.0 | 239.9 | 195.9 | 287.2 | 1.466 | 0.3 | 1.9 |
| | 2 | 44.0 | 237.6 | 193.6 | 292.5 | 1.511 | -0.9 | -1.1 |
| | 5 | 44.0 | 246.5 | 202.5 | 297.5 | 1.469 | 3.6 | 1.7 |
| | 10 | 44.0 | 246.8 | 202.8 | 292.0 | 1.440 | 3.8 | 3.7 |
| | 20 | 44.0 | 257.7 | 213.8 | 303.4 | 1.419 | 9.4 | 5.1 |
| | 30 | 44.0 | 248.2 | 204.3 | 293.4 | 1.436 | 4.6 | 3.9 |
| Antibiotic trans-BM123γ Tru-Tan® | 1 | 44.1 | 245.2 | 201.2 | 294.0 | 1.461 | 3.0 | 2.3 |
| | 2 | 44.0 | 244.4 | 200.4 | 296.2 | 1.478 | 2.6 | 1.1 |
| | 5 | 44.0 | 249.4 | 205.4 | 292.9 | 1.426 | 5.1 | 4.6 |
| | 10 | 44.0 | 251.8 | 207.8 | 299.6 | 1.442 | 6.3 | 3.5 |
| | 20 | 44.0 | 251.8 | 207.8 | 302.1 | 1.454 | 6.3 | 2.7 |
| | 30 | 44.0 | 248.1 | 204.1 | 294.9 | 1.445 | 4.5 | 3.3 |

TABLE II

| Treatment | Drug Level In Feed (ppm) | Av. Weight per Chick In Grams | | Gain Per Chick In Grams | Feed Consumed Per Chick In Grams (Average) | Feed/ Gain Ratio | % Improvement In | |
|---|---|---|---|---|---|---|---|---|
| | | Start | End | | | | Gain | Feed/Gain Ratio |
| Control | | 41.3 | 284.1 | 242.8 | 338.0 | 1.392 | | |
| Antibiotic trans- | 5 | 41.3 | 290.2 | 248.9 | 341.0 | 1.370 | 2.5 | 1.5 |
| BM123γ Dioctyl | 10 | 41.3 | 302.3 | 261.0 | 346.9 | 1.329 | 7.5 | 4.5 |
| Sulfosuccinate | 20 | 41.3 | 297.1 | 255.8 | 343.0 | 1.341 | 5.3 | 3.7 |
| Complex Precipitated | 40 | 41.3 | 296.2 | 254.9 | 344.6 | 1.352 | 5.0 | 2.9 |
| from Fermentation Mash Filtrate | | | | | | | | |

## TABLE III

Evaluation of the Efficacy of Trans-BM123γ-hydrochloride as a Feed Additive for the Enhancement of the Growth Rate and for increasing Feed Efficiency of Broilers fed for 13 days.

| Treatment | Drug Level in Feed (ppm) | Average weight per Chick in Gram | | Average Weight gain per Chick in Gram | Feed Consumed per Chick in Gram (Average) | Feed/ Gain Ratio | Percent Improvement in | |
|---|---|---|---|---|---|---|---|---|
| | | Start | End | | | | Gain | Feed/Gain Ratio |
| Control | 0 | 38.8 | 211.7 | 172.9 | 273.7 | 1.583 | | |
| Trans- BM123γ- hydrochloride mixture | 10 | 38.8 | 231.5 | 192.7 | 277.5 | 1.440 | 11.5 | 9.9 |
| | 20 | 38.8 | 226.1 | 187.3 | 281.6 | 1.503 | 8.3 | 5.3 |
| | 40 | 38.7 | 227.2 | 188.4 | 275.4 | 1.462 | 9.0 | 8.3 |

TABLE IV

Evaluation of the Efficacy of Trans-BM123γ-hydrochloride as a Feed Additive for the Enhancement of the Growth Rate and for Improving Feed Efficiency of Broilers fed for 28 days.

| Treatment | Drug Level in Feed (ppm) | Average weight per Chick in Gram | | Average weight gain per Chick in Gram | Feed Consumed per Chick in Gram (Average) | Feed/Gain Ratio | Percent Improvement in | |
|---|---|---|---|---|---|---|---|---|
| | | Start | End | | | | Gain | Feed/Gain Ratio |
| Control | 0 | 42.8 | 767.5 | 722.5 | 1119.9 | 1.550 | | |
| Trans-BM123γ hydrochloride | 1 | 42.8 | 757.3 | 714.5 | 1095.3 | 1.533 | -1.1 | 1.1 |
| | 2 | 42.8 | 787.2 | 744.4 | 1111.4 | 1.493 | 3.0 | 3.7 |
| | 5 | 42.8 | 785.8 | 743.0 | 1116.0 | 1.502 | 2.8 | 3.1 |
| | 10 | 42.7 | 809.0 | 766.2 | 1155.4 | 1.508 | 6.1 | 2.7 |
| | 20 | 42.8 | 795.6 | 752.7 | 1128.3 | 1.499 | 4.2 | 3.3 |

Example 8

Growth promoting effect of antibiotic BM123γ- alkyl Sulfate complex on weanling pigs

Forty, five year old weanling pigs are allotted randomly into four groups of ten pigs each. One group serves as unmedicated controls. The other three groups receive antiboitic BM123γ - lauryl sulfate complex in their diets at levels of 25, 50 and 100 ppm. Control animals are offered a modified pig starter ration (composition follows) and water ad libitum. The other groups receive the same diet except that it contains the aforementioned complex at the levels indicated.

The weight of the pigs is determined at the start of the experiment and two weeks later. Average daily weight gains, average overall weight gains, weight of food consumed per pen, feed/gain ratios and the percent improvements of weight gains and feed/grain ratios over controls are also determined and appear in Table V below.

Modified pig starter ration

| Composition | lb./ton |
|---|---|
| Corn | 1250 |
| Farm blend protein supplement* | 600 |
| Dried whey | 150 |

*Farm blend protein supplement

Processed grain by-products, animal protein products, plant protein products, cane molasses, forage products, vitamin A supplement, D activated animal sterol, vitamin $B_{12}$ supplement, vitamin E supplement, riboflavin supplement, methionine hydroxy analog calcium,

niacin, biotin, choline chloride, calcium pantothenate, defluorinated phosphate, calcium carbonate, iodized salt, sodium selenide, iron carbonate, iron sulfate, manganous oxide, copper sulfate, cobalt carbonate, zinc oxide.

Analysis

| | | |
|---|---|---|
| Crude protein | ≧ | 36.0% |
| Crude fat | ≧ | 0.5% |
| Crude fiber | ≦ | 7.0% |
| Calcium | ≧ | 3.2% |
| Calcium | ≦ | 4.2% |
| Phosphorus | ≧ | 1.7% |
| Iodine | ≧ | 0.0003% |
| Sodium chloride | ≧ | 2.3% |
| Sodium chloride | ≦ | 3.3% |

TABLE V

Efficacy of *trans*-BM123γ-sodium lauryl sulfate complex in improving the growth rate and feed efficiency in weanling pigs. Two weeks feeding data, averaged.

| Group | Drug level in Diet (ppm) | Average Weight per pig in kg | | Average weight gain per pig in kg | | % Improvement over Control | Total Feed per pen in kg | Feed/Gain Ratio | % Improvement over control |
|---|---|---|---|---|---|---|---|---|---|
| | | Start | Finish | Two Weeks | Daily | | | | |
| Control | 0 | 8.45 | 13.55 | 5.13 | 0.366 | | 95.2 | 1.855 | |
| 1 | 25 | 8.31 | 14.95 | 6.66 | 0.476 | 30 | 114.8 | 1.768 | 5 |
| 2 | 50 | 8.15 | 14.70 | 6.57 | 0.469 | 28 | 103.3 | 1.572 | 15 |
| 3 | 100 | 8.52 | 15.80 | 7.3 | 0.521 | 42 | 117.8 | 1.613 | 13 |

Example 9

Evaluation of antibiotic trans-BM123γ-hydrochloride as a feed additive for the enhancement of the growth rate of poultry.

Test Drugs

1. Antibiotic trans-BM123γ-hydrochloride, purified.

2. Antibiotic trans-BM123γ-hydrochloride, obtained by spray-drying the as is fermentation mash.

The above drugs are administered to the test animals in their feed at the levels indicated in Table IV below.

Test Animals

One day old Hubbard X Hubbard Crossbred chicks, randomly allotted to pens of ten chicks (5 males and 5 females) each.

Procedure

The procedure of Example 7 is used, except that two pens of chicks are used at each level of drug. Thus in this set of experiments a total of 16 pens (160 chicks) are used as controls and a total of 8 pens (80 chicks) are used at each level of drug. The duration of each experiment is 13 days.

The data obtained are averaged and summarized in Table VI below, wherein the percent improvement in weight gains, and in feed/gain ratios is also given.

TABLE VI

Evaluation of the Efficacy of Antibiotic trans-BM123γ-hydrochloride samples, obtained by different methods, for the Enhancement of the Growth Rate of Poultry and for Improving Feed Efficiency.

| Treatment | Drug Level in Feed (ppm) | Average weight per Chick in Gram | | Average weight gain per Chick in Gram | Feed Consumed per Chick in Gram (Average) | Feed/ Gain Ratio | Percent Improvement in | |
|---|---|---|---|---|---|---|---|---|
| | | Start | End | | | | Gain | Feed/Gain Ratio |
| Control | 0 | 43.7 | 234.1 | 190.4 | 294.4 | 1.546 | | |
| Antibiotic trans-BM123γ hydrochloride | 2 | 43.7 | 229.9 | 186.2 | 288.2 | 1.548 | -2.2 | -0.1 |
| | 5 | 43.7 | 238.4 | 194.7 | 293.0 | 1.505 | 2.3 | 2.7 |
| | 10 | 43.7 | 242.2 | 198.5 | 295.2 | 1.487 | 4.3 | 3.8 |
| | 20 | 43.8 | 245.3 | 201.6 | 297.2 | 1.474 | 5.9 | 4.7 |
| Antibiotic * trans-BM123γ hydrochloride | 1 | 43.8 | 233.6 | 189.9 | 291.5 | 1.535 | -0.3 | 0.7 |
| | 2 | 43.7 | 236.1 | 192.4 | 291.5 | 1.515 | 1.1 | 2.0 |
| | 5 | 43.8 | 245.0 | 201.3 | 296.9 | 1.475 | 5.7 | 4.6 |
| | 10 | 43.7 | 249.7 | 206.0 | 295.0 | 1.432 | 8.2 | 7.4 |
| | 20 | 43.7 | 251.9 | 208.2 | 301.9 | 1.450 | 9.3 | 6.2 |

*Spray-dried fermentation mash.

## Example 10

Evaluation of antibiotic trans-BM123$\gamma$-hydrochloride as feed additives for the enhancement of the growth rate of poultry.

Test Drug

Sample # 1. Antibiotic trans-BM123 -hydrochloride, low$\gamma_2$ $\gamma_2$ content.

Sample # 2. Antibiotic trans-BM123$\gamma$-hydrochloride, high $\gamma_2$ content.

The drug samples are administered to the test animals in their feed at the levels indicated in Table VII below.

Test Animals

One day old Hubbard X Hubbard crossbred chicks, randomly allotted to pens of ten chicks (5 males and 5 females) each.

Procedure

The procedure of Example 7 is used. The duration of each experiment is 14 days.

The data obtained are averaged and summarized in Table VII below, wherein the percent improvement is weight gains, and in feed/gain ratios is also given.

TABLE VII

Evaluation of the Efficacy of antibiotic trans-BM123γ-hydrochloride samples of various isomeric composition, for the Enhancement of the growth rate of poultry, and for Improving Feed Efficiency.

| Treatment | Drug Level in Feed (ppm) | Average weight per Chick in Gram | | Average gain weight gain per Chick in Gram | Feed Consumed per Chick in Gram (Average) | Feed/ Gain Ratio | Percent Improvement in | |
|---|---|---|---|---|---|---|---|---|
| | | Start | End | | | | Gain | Feed/Gain Ratio |
| Control | 0 | 42.4 | 271.5 | 229.1 | 342.0 | 1.493 | | |
| Antibiotic trans-BM123γ hydrochloride Sample #1 | 10 | 42.4 | 279.9 | 237.4 | 344.5 | 1.451 | 3.6 | 2.8 |
| | 20 | 42.4 | 282.0 | 239.7 | 347.6 | 1.450 | 4.6 | 2.9 |
| Antibiotic trans-BM123γ hydrochloride Sample # 2 | 5 | 42.3 | 275.9 | 233.6 | 341.5 | 1.462 | 2.0 | 2.1 |
| | 10 | 42.3 | 274.1 | 231.8 | 332.2 | 1.433 | 1.2 | 4.0 |
| | 20 | 42.4 | 285.8 | 243.5 | 344.8 | 1.416 | 6.3 | 5.2 |

Example 11

Evaluation of the efficacy of trans-BM123γ sodium lauryl sulfate complex in improving the growth rate and feed efficiency of weanling pigs

Test Animals

Forty, five week old weanling pigs are allotted randomly into 4 groups of 10 pigs each. One group serves as unmedicated controls. The other groups receive the drug in their diet at the levels indicated in Table VIII below.

Procedure

Control animals are offered a modified pig starter ration (composition of same is appended hereto) and water *ad libitum*. The other groups receive the same diet except that it is medicated with the test drug at the levels indicated.

The weight of the pigs is determined at the start and two weeks later. Further, average daily weight gains, average overall weight gains, weight of feed consumed per pen, feed/gain ratios, and the percent improvements (over controls) of weight gains and feed/gain ratios are also determined. The thus obtained data are summarized in Table VIII below.

**Modified Pig Starter Ration**

| Composition | lb./ton |
|---|---|
| Corn | 1250 |
| Farm Blend Protein Supplement* | 600 |
| Dried whey | 150 |
| | 2000 |

* Farm Blend Protein Supplement

Ingredients

Processed grain by-products, animal protein products, plant protein products, cane molasses, forage products, vitamin A supplement, D activated animal sterol, vitamin B12 supplement, vitamin E supplement, riboflavin supplement, methionine hydroxy analogue calcium, niacin, biotin, choline chloride, calcium pantothenate, defluorinated phosphate, calcium carbonate, iodized salt, sodium selenide, iron carbonate, iron sulfate, manganous oxide, copper sulfate, cobalt carbonate, zinc oxide.

Analysis

| | |
|---|---|
| Crude protein not less than | 36.0% |
| Crude fat not less than | 0.5 |
| Crude fiber not more than | 7.0 |
| Calcium (Ca) not less than | 3.2 |
| Calcium (Ca) not more than | 4.2 |
| Phosphorus (P) not less than | 1.7 |
| Iodine not less than | 0.0003 |
| Salt (NaCl) not less than | 2.3 |
| Salt (NaCl) not more than | 3.3 |

TABLE VIII

Efficacy of *trans*-BM123γ-sodium lauryl sulfate complexed in improving the growth rate and feed efficiency in weanling pigs. Two weeks feeding data, averaged.

| Group | Drug level in Diet (ppm) | Average Weight per pig in kg | | Average Weight gain per pig in kg | | % Improvement over Control | Total Feed per pen in kg | Feed/ Gain Ratio | % Improvement over control |
|---|---|---|---|---|---|---|---|---|---|
| | | Start | Finish | Two Weeks | Daily | | | | |
| Control | 0 | 8.45 | 13.55 | 5.13 | 0.366 | | 95.2 | 1.855 | |
| 1 | 25 | 8.31 | 14.95 | 6.66 | 0.476 | 30 | 114.8 | 1.768 | 5 |
| 2 | 50 | 8.15 | 14.70 | 6.57 | 0.469 | 28 | 103.3 | 1.572 | 15 |
| 3 | 100 | 8.52 | 15.80 | 7.3 | 0.521 | 42 | 117.8 | 1.613 | 13 |

## Example 12

Evaluation of certain trans-BM123γ-alkyl derivatives as feed additives for the enhancement of the growth rate of poultry.

Test Drugs

#1. Antibiotic trans-BM123γ-HCl (60%)

#2. Antibiotic trans-BM123γ-HCl spray dried filtrate (0.933%).

# 3. Antibiotic trans-BM123γ -sodium lauryl sulfate (0.447%).

# 4. Isopropyl-trans-BM123γ.

# 5. 1-Methylnonyl-trans-BM123γ.

# 6. 1,3-Dimethylbutyl-trans-BM123γ.

# 7. 1,1,3-Trimethylbutyl-trans-BM123γ.

# 8. 1-Ethyl-3-chloropropyl-trans-BM123γ.

# 9. 1,2-Dimethylpentyl-trans-BM123γ.

#10. 1-Methyl-2-hydroxypropyl-trans-BM123γ.

Test Animals

One day old Hubbard X Hubbard crossbred chicks, randomly allotted to pens of ten chicks (5 males and 5 females) each.

Procedure

The procedure of Example 7 is used. The duration of each experiment is 14 days.

The data obtained are averaged and summarized in Table IX below.

## TABLE IX

Evaluation of the Efficacy of Certain BM123γ-alkyl derivatives for the Enhancement of the Growth Rate of Poultry and for Improving Feed Efficiency.

| Treatment | Drug Level in Feed (ppm) | Average Weight per Chick in Gram | | Average Weight gain per Chick in Gram | Feed Consumed per Chick in gram (Average) | Feed/Gain Ratio | Percent Improvement in | |
|---|---|---|---|---|---|---|---|---|
| | | Start | End | | | | Gain | Feed/Gain Ratio |
| Control | 0 | 37.3 | 272.5 | 235.2 | 329.5 | 1.401 | | |
| #1 | 5 | 37.3 | 276.7 | 239.4 | 333.9 | 1.395 | 1.78 | 0.43 |
| | 10 | 37.3 | 280.6 | 243.3 | 328.7 | 1.351 | 3.74 | 3.57 |
| | 20 | 37.3 | 291.4 | 254.1 | 346.2 | 1.363 | 8.04 | 2.71 |
| #2 | 5 | 37.3 | 285.4 | 248.1 | 334.9 | 1.350 | 5.48 | 3.64 |
| | 10 | 37.3 | 284.3 | 247.0 | 332.6 | 1.347 | 5.02 | 3.85 |
| | 20 | 37.3 | 292.1 | 254.8 | 340.0 | 1.334 | 8.33 | 4.78 |
| #3 | 10 | 37.3 | 288.2 | 250.9 | 328.6 | 1.310 | 6.68 | 6.50 |
| #4 | 5 | 37.3 | 281.8 | 244.5 | 331.4 | 1.355 | 3.95 | 3.28 |
| | 10 | 37.3 | 283.1 | 245.8 | 328.2 | 1.335 | 4.51 | 4.71 |
| #5 | 10 | 37.3 | 282.6 | 245.3 | 334.0 | 1.362 | 4.29 | 2.78 |
| #6 | 10 | 37.3 | 283.8 | 246.5 | 334.1 | 1.358 | 4.80 | 3.07 |
| #7 | 10 | 37.3 | 282.2 | 244.9 | 333.4 | 1.361 | 4.12 | 2.86 |
| #8 | 5 | 37.3 | 276.6 | 239.3 | 328.9 | 1.375 | 1.74 | 1.86 |
| #9 | 10 | 37.3 | 279.8 | 242.5 | 334.7 | 1.380 | 3.10 | 1.50 |
| #10 | 10 | 37.3 | 280.4 | 243.1 | 331.5 | 1.364 | 3.76 | 2.64 |

0003644

CLAIMS

1. A process for the production of an animal feed supplement, characterized by adding to a fermentation whole harvest mash containing antibiotic trans-BM123γ, or to a filtered fermentation broth containing antibiotic BM 123γ, at a suitable acid pH, a complexing agent selected from synthetic tanning agents, alkali metal alkyl sulfates and dioctyl sulfosuccinate salts, whereby to precipitate out a complex of the antibiotic with said complexing agent; and recovering the precipitated complex.

2. A process according to Claim 1 for the production of a dried fermentation harvest mash solids animal feed supplement containing an antibiotic trans-BM123 -alkyl sulfate complex which comprises the steps of:

(a) acidifying a fermentation whole harvest mash containing antibiotic trans-BM123 to a pH of from 1.9 to 2.1;

(b) adding to the acidified mash, in amount sufficient to produce a complex with the antibiotic trans--BM123 , a complexing agent selected from the group consisting of compounds of the formula:

$$CH_3-(CH_2)_n-O-SO_2-OM$$

wherein n is an integer from 9 to 17, inclusive, and M is sodium or potassium, and mixtures thereof;

(c) removing the harvest mash solids together with the precipitated antibiotic trans-BM123 -alkyl sulfate complex; and

(d) drying the mixture of mash solids and antibiotic trans-BM123 -alkyl sulfate complex.

3. A process according to Claim 2, wherein the complexing agent is sodium decyl sulfate, sodium lauryl

sulfate, or a mixture of sodium cetyl sulfate and sodium oleyl sulfate.

4. A process according to Claim 1 for the production of a dried fermentation harvest mash solids animal feed supplement containing an antibiotic trans-BM-123 -dioctyl sulfosuccinate complex which comprises the steps of:

(a) acidifying a fermentation whole harvest mash containing antibiotic trans-BM123 to a pH from 2.5 to 6.0 with a pharmacologically acceptable acid;

(b) adding to the acidified mash, in amount sufficient to produce a complex with the antibiotic trans-BM123 , a complexing agent selected from the group consisting of the alkali metal and alkaline earth metal salts of sulfobutanedioic acid 1,4-bis(2-ethylhexyl)ester and mixtures thereof;

(c) removing the harvest mash solids together with the precipitated antibiotic trans-BM123 - dioctyl sulfosuccinate complex; and

(d) drying the mixture of mash solids and antiobiotic trans-BM123 - dioctyl sulfosuccinate complex.

5. A process according to Claim 4, wherein the complexing agent is dioctyl potassium sulfosuccinate or dioctyl magnesium sulfosuccinate.

6. A process according to Claim 1 for the production of a dried fermentation harvest mash solids animal feed supplement containing an antibiotic trans-BM123 -syntan complex which comprises the steps of:

a. adding to the fermentation whole harvest mash a syntan complexing agent comprising a mixture of compounds of the formula:

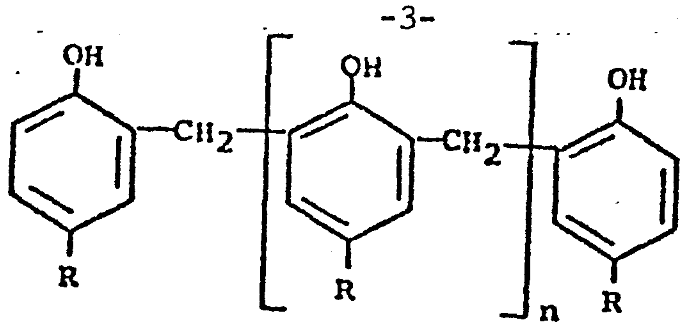

wherein R is hydrogen or methylene sulfonic acid and n is 0, 1, 2, 3, or 4 with the proviso that about half of the R's present are methylene sulfonic acid, until a sufficient amount of the antibiotic trans-BM123 -syntan complex is imparted to said medium;

b. adjusting the medium to a pH of from 1.8 to 5.0 with a pharmacologically acceptable acid;

c. removing the harvest mash solids together with the precipitated antibiotic trans-BM123 - syntan complex; and

d. drying the mixture of mash solids and antibiotic trans-BM123 - syntan complex.

7. An animal feed supplement containing an antibiotic trans-BM123 - complex, whenever produced by a process as defined in any one of the preceding claims.

8. An animal feed composition for improving feed efficiency and enhancing the growth rate of animals and poultry characterized as a nutritionally balanced animal feed containing from about 0.0001% to about 1.0% by weight of the feed of an antibacterial ingredient selected from the group consisting of antibiotic trans-BM123 , an antibiotic trans-BM123 salt, an antibiotic trans-BM123 alkyl sulfate complex, an antibiotic trans-BM123 dioctyl sulfosuccinate complex, an antibiotic trans-BM123 syntan complex, an antibiotic trans-BM123 alkylated derivative, and mixtures thereof in any proportion.

9. An animal feed supplement characterized as a dried mixture of fermentation harvest mash solids and an antibacterial ingredient selected from the group consisting of antibiotic trans-BM123 , an antibiotic trans-BM123 salt, an antibiotic trans-BM123 alkyl sulfate complex, an antibiotic trans-BM123 dioctyl sulfosuccinate complex, an antibiotic trans-BM123 syntan complex, an antiobiotic trans-BM123 alkylated derivative, and mixtures thereof in any proportion.

European Patent Office

# EUROPEAN SEARCH REPORT

0003644

Application number

EP 79 30 0116

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| D | FR - A - 2 202 680 (CYANAMID)<br>* Complete specification *<br>& US - A - 4 007 167<br>-- | 1,8 | C 12 D 9/00<br>C 12 D 9/14<br>C 07 H 15/20<br>C 07 G 11/00<br>C 07 H 15/22<br>A 23 K 1/17 |
| | US - A - 3 856 937 (CYANAMID)<br>* Complete specification *<br>-- | 1,2,3,7 | |
| | US - A - 3 832 462 (CYANAMID)<br>* Complete specification *<br>---- | 1,6,7 | TECHNICAL FIELDS SEARCHED (Int.Cl.²)<br>C 12 D 9/00<br>9/14<br>C 07 H 15/20<br>C 07 G 11/00<br>C 07 H 15/22<br>A 23 K 1/17 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| ✓ | The present search report has been drawn up for all claims | | &: member of the same patent family, corresponding document |

| Place of search | Date of completion of the search | Examiner |
|---|---|---|
| The Hague | 10-05-1979 | RAJIC |

EPO Form 1503.1 06.78